(19)

**Europäisches Patentamt**
**European Patent Office**
**Office européen des brevets**

(11)  **EP 3 292 534 B1**

(12)  **EUROPEAN PATENT SPECIFICATION**

(45) Date of publication and mention
of the grant of the patent:
**23.07.2025   Bulletin 2025/30**

(21) Application number: **16789682.8**

(22) Date of filing: **29.04.2016**

(51) International Patent Classification (IPC):
$G16H\ 10/20^{(2018.01)}$   $G16H\ 10/40^{(2018.01)}$
$G16H\ 20/60^{(2018.01)}$   $G16H\ 20/70^{(2018.01)}$
$G16H\ 40/67^{(2018.01)}$   $G16H\ 50/30^{(2018.01)}$
$A61B\ 5/18^{(2006.01)}$   $A61B\ 5/16^{(2006.01)}$
$A61B\ 5/00^{(2006.01)}$   $G06Q\ 10/06^{(2023.01)}$
$G06Q\ 10/0639^{(2023.01)}$   $G06Q\ 50/22^{(2024.01)}$

(52) Cooperative Patent Classification (CPC):
**A61B 5/165; A61B 5/18; A61B 5/4845;**
**A61B 5/7275; G06Q 10/0639; G16H 10/20;**
**G16H 10/40; G16H 20/60; G16H 20/70;**
**G16H 40/67; G16H 50/30**

(86) International application number:
**PCT/SE2016/050380**

(87) International publication number:
**WO 2016/178617 (10.11.2016 Gazette 2016/45)**

(54) **METHOD AND DEVICE FOR ESTIMATING A RISK OF RELAPSE OF ADDICTIVE BEHAVIOUR**

VERFAHREN UND VORRICHTUNG ZUR SCHÄTZUNG EINES RISIKOS DES RÜCKFALLS EINES
SUCHTVERHALTENS

PROCÉDÉ ET DISPOSITIF D'ESTIMATION DU RISQUE DE RECHUTE D'UN COMPORTEMENT
ADDICTIF

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:  **04.05.2015   SE 1500215**

(43) Date of publication of application:
**14.03.2018   Bulletin 2018/11**

(73) Proprietor: **Kontigo Care AB**
**753 20 Uppsala (SE)**

(72) Inventors:
• **HÄMÄLÄINEN, Markku**
**753 30 Uppsala (SE)**

• **ANDERSSON, Karl**
**755 78 Vänge (SE)**

(74) Representative: **AWA Sweden AB**
**Box 5117**
**200 71 Malmö (SE)**

(56) References cited:
**WO-A1-2014/127234**    **WO-A1-2015/021042**
**WO-A1-2015/047110**    **WO-A2-2006/122325**
**US-A1- 2003 212 579**    **US-A1- 2007 100 216**
**US-A1- 2009 070 139**    **US-A1- 2013 166 319**
**US-A1- 2014 052 474**

## Description

### Field of invention

The present invention generally relates to addictive disorders and more specifically to methods and devices for estimating a risk of relapse of addictive behavior.

### Background of the invention

Addictive disorders are common in modern society. Some individuals are addicted to alcohol or other drugs (cocaine, heroin, methamphetamine to mention a few non-limiting examples). Some other individuals are addicted to food consumption and still others are addicted to gaming of various kinds. Smoking is also an addictive behavior that sometimes is seen as a disorder.

Addictive disorders pose a serious threat to modern society. Addiction can take many forms, and one common form of addiction is the intentional use of chemical compounds such as alcohol that intoxicate the user, which causes high costs for health care. Another possible addiction is food related, where a user is either eating excessive resulting in obesity or starving resulting in anorexia. Yet another possible addiction is related to computer gaming, where a user is spending an unhealthy amount of time on computer based games with the potential result of ruining private economy. There are treatment programs for these and other addictive disorders.

One approach to estimate risk for relapse of alcohol dependence has been described in the patent application UA79063 "Method for integrated diagnostics of possibilities of alcohol dependence relapses". The document discloses a method for integrated diagnostics of possibilities of relapse which contains determination of specific and symptomatic syndromologic signs of alcohol dependence. In addition, test monitoring of general psychological quality of life and diagnosis of latent tremor by a laser scanning method is performed. Output of measurements are combined into a score which is indicative of risk for relapse.

Gaming and gambling disorders have relapse patterns that are difficult to manage. One exemplary disclosure on this subject is the report "Retrospective and Prospective Reports of Precipitants to Relapse in Pathological Gambling." by Hodgins, David C. and el-Guebaly, Nady as published in Journal of Consulting and Clinical Psychology, Vol 72(1), Feb 2004, 72-80. http://dx.doi.org/10.1037/0022-006X.72.1.72. This document discloses that before a gaming disorder relapse, participants in the clinical study reported a wide range of moods and emotions with no dominant pattern. Furthermore, men and women also attributed relapses to different causes. The suggestion is that there is no quick fix or single treatment model for gambling relapse. Interestingly, reports of positive moods were as common as negative moods among the participants. This finding stands, according to the authors, in contrast to previous findings that negative moods are the most frequent predictor across a range of addictive behaviors. Another contradictory finding was reported: The reasons for quitting gambling are similar to the reasons for relapse to some degree. All in all, this disclosure describes that the understanding of gaming disorder is still limited, which leads to the conclusion that treatment programs and relapse monitoring have great room for improvement.

In the disclosure "Predictive Modeling of Addiction Lapses in a Mobile Health Application" by Chih and co-authors (as published in J Subst Abuse Treat. 2014 January ; 46(1): 29-35.

doi:10.1016/j.jsat.2013.08.004.), a questionnaire-based system is used to predict and reduce relapses of addictive behavior. This is a weekly check-up system suitable for the monitoring of recovery from addiction to alcohol.

In the published international patent application WO 2015/021042 A1, a system and method for managing binge eating disorders (BED) is disclosed. A smartphone and server application is provided for monitoring and treating BED. Ecological Momentary Assessment (EMA) may be used for automated and/or manual acquisition of data related to triggers associated with BED. Risk factors may be captured in real-time to provide data to both patients and clinicians. For manual data acquisition, the user may be prompted to input emotional states, urges to binge, eating behavior, and binge episodes. For automated acquisition, time, place (e.g. via GPS), medicine adherence, and physical activity may be recorded. The user may be provided with alerts and/ or interventions when it is determined that they are at risk of binging.

### Summary of the invention

It is an object of the present disclosure to provide methods, devices and a computer program product for estimating a risk of relapse of addictive behavior. This and other objects are met by embodiments of the invention disclosed in the present application, which is based on the repeated registering and analysis of exposure to stimuli which results in a capability to provide a more complete view of an individual's exposure to stimuli when estimating the risk for relapse, for example during or after treatment of addictive behavior related to said stimuli.

Accordingly, based on the discoveries of the present disclosure, a first aspect of the present disclosure provides a method for estimating a risk of relapse of addictive behavior of an individual, said addictive behavior being related to exposure to a predefined addictive stimulus, the method comprising the steps of, at multiple points in time, repeatedly, requesting, by a central server unit, the individual to do at least one of conducting a measurement of the individual's exposure to the stimulus and answering a questionnaire provided in a measurement device; performing at least one of measuring, with the measurement device, the individual's exposure to the stimulus and collecting, with the measurement device, the individual's questionnaire answers; transmitting a result from the stimulus exposure measurement, if performed, and the questionnaire answers, if collected, from the measurement device to a central server unit; storing the result in the central server unit; upon the central server unit receiving the result, the central server unit estimating a risk of relapse based on a combination of the received result and historic results, and at least one of the following; time of day when measuring the individual's exposure to the stimulus; day of week when measuring the individual's exposure to the stimulus; result of previous measurement of the individual's exposure to the stimulus; elapsed time since previous measurement of the individual's exposure to the stimulus; number of historic measurements of the individual's exposure to the stimulus that have been missed; determining, by the central server unit, a next point in time for requesting the individual to do at least one of conducting a measurement of the individual's exposure to the stimulus and answering a questionnaire, based on the estimated risk, wherein the next point in time is determined so as to collect a time-line of stimuli exposure data that reveals onset patterns, frequency, duration and intensity of stimuli exposure; and if the estimated risk is greater than a predefined level, transmitting, from the central server unit, a message to a third party wherein said third party is at least one of a health care provider, a family member or close relative, an employer, a law enforcement organization, and a governmental institution.

A second aspect of the present disclosure provides a server unit configured to estimate a risk of relapse of addictive behavior of an individual, the addictive behavior being related to exposure to an addictive stimulus, wherein the server unit is configured to, at multiple points in time, repeatedly, request an individual to do at least one of conducting a measurement of the individual's exposure to the stimulus and answering a questionnaire provided in a measurement device; receive a result from the stimulus exposure measurement, if performed, and the questionnaire answers, if collected, from the measurement device; store the received result; upon receiving the result, estimate a risk of relapse based on a combination of the received result and historic results and at least one of the following; time of day when measuring the individual's exposure to the stimulus; day of week when measuring the individual's exposure to the stimulus; result of previous measurement of the individual's exposure to the stimulus; elapsed time since previous measurement of the individual's exposure to the stimulus; number of historic measurements of the individual's exposure to the stimulus that have been missed; determine a next point in time for requesting the individual to do at least one of conducting a measurement of the individual's exposure to the stimulus and answering a questionnaire, based on the estimated risk, wherein the next point in time is determined so as to collect a time-line of stimuli exposure data that reveals onset patterns, frequency, duration and intensity of stimuli exposure; and if the estimated risk is greater than a predefined level, transmit a message to a third party, wherein said third party is at least one of a health care provider, a family member or close relative, an employer, a law enforcement organization, and a governmental institution.

A third aspect of the present disclosure provides a system configured to estimate a risk of relapse of addictive behavior of an individual, the addictive behavior being related to exposure to an addictive stimulus, wherein the system comprises a server unit according to the second aspect, and a measurement device configured to, at multiple points in time, perform at least one of measuring the individual's exposure to the stimulus, providing a questionnaire to the individual and collecting the individual's questionnaire answers, transmit a result from the stimulus exposure measurement, if performed, and the questionnaire answers, if collected, to a central server unit to enable estimation of a risk of relapse, and wherein the measurement device is further configured to enable identification of the individual during measurement of the individual's exposure to the stimulus.

A fourth aspect of the present disclosure provides a computer-program product comprising a computer-readable medium having stored thereon a computer program comprising instructions, which when executed by at least one processor, cause the at least one processor to perform steps of the method of the first aspect.

## Brief description of the drawings

The embodiments, together with further objects and advantages thereof, may best be understood by making reference to the following description taken together with the accompanying drawings, in which:

Figure 1 illustrates an example of a multiple choice question as part of a digital questionnaire.

Figure 2a is a schematic flow diagram illustrating an example of a method for estimating a risk of relapse of addictive behavior according to an embodiment.

Figure 2b is a schematic flow diagram illustrating optional additional steps of the method of Figure 2a.

Figure 3 is a schematic diagram illustrating an example of a system configured to estimate a risk of relapse of addictive behavior according to an embodiment.

Figure 4 shows the sobriety index plotted versus time for an individual who was in treatment for alcohol abuse according to an example.

Figure 5 shows the prediction of sobriety index plotted versus actual sobriety index for 12 individuals according to an example.

Figure 6 illustrates the construction of sobriety index for three different individuals according to an example.

Figure 7 shows a mood adjusted sobriety index for two different individuals according to an example.

## Detailed description of the invention

For the purpose of this application and for clarity, the following definitions are made:
The term "addiction" refers to a state of an individual characterized by compulsive engagement in rewarding stimuli (i.e. stimuli that the brain interprets as intrinsically positive or as something to be approached), despite adverse consequences.
The term "addictive behavior" refers to a behavior which is both rewarding and reinforcing. It may involve any activity, substance, object, or behavior that becomes the major focus of an individual's life and which results in a physical, mental, and/or social withdrawal from normal day to day obligations.
The term "stimulus" refers to an activity, or substance that an individual is addicted to, and that may cause addictive behavior. One non-limiting example of a stimulus is gambling. Another non-limiting stimulus is food and eating. Yet another non-limiting example of a stimulus is an intoxicating chemical compound.
The term "intoxicating chemical compound" refers to a single compound or a combination of multiple compounds capable of intoxicating an individual to a level where the general status of said individual is affected. One non-limiting example of an intoxicating chemical compound is ethanol, more commonly known as alcohol, which is readily available to individuals in wine, beer, spirits and other beverages. Ethanol is intoxicating individuals to a level where many countries have a limit for the allowed amount of ethanol in the blood to drive a car legally. Other intoxicating chemical compounds include, but are not limited to: cannabinoids as for example available in cannabis, caffeine, MDMA (3,4-methylenedioxy-methamphetamine), cocaine, amphetamine, methamphetamine, psilocybin (for example found in "magic mushrooms"), LSD, opiates and opioids, tranquilizers like barbiturates, benzodiazepines and the similar, ketamine, amyl nitrite, mephedrone, mescaline, DMT for example as primary ingredient in ayahuasca, cathine and cathinone (khat), methylphenidate, fentanyl, GHB, ecstasy, narcolepsy medications, sleeping pills, anxiolytics, sedatives, cough suppressants, benzydamine, ephedrine, pseudoephedrine, dimethyltryptamine (DMT), 5-MeO-DMT, theobromine, kavalactones, myristicin, atropine, scopolamine, mitragynine, mitraphylline, 7-hydroxymitragynine, raubasine, valerian, lysergic acid amide (LSA, ergine), ibogaine, arecoline, rauwolscine, yohimbine, corynantheidine, psilocybin, psilocin, bufotenin, ibotenic acid, muscimol, antihistamines including but not limited to diphenhydramine, chlorpheniramine, orphenadrine and hydroxyzine, scopolamine, paracetamol (para-acetylaminophenol), non-steroidal anti-inflammatory drugs (NSAIDs) such as salicylates, hydrocodone, codeine, oxycodone, hydromorphone, carisoprodol, chloral hydrate, diethyl ether, ethchlorvynol, gabapentin, gamma-butyrolactone (GBL, a prodrug to GHB), gamma-hydroxybutyrate (GHB), glutethimide, ketamine, meprobamate, methaqualone, phenibut, pregabalin, propofol, nepetalactone, dimenhydrinate, hyoscyamine, dextromethorphan, dextromethorphan, chlorpheniramine, methoxetamine, phencyclidine, and nitrous oxide, to mention a few examples.
The term "stimuli exposure" refers to an individual becoming exposed to a particular stimulus, either through own will or unintentionally. For example, in cases where the stimulus is an activity such as gambling or eating, "stimuli exposure" refers to engaging in such an activity. As another example, in cases where the stimulus is an intoxicating chemical compound, "stimuli exposure" refers to ingesting, injecting, inhaling, or in any other way introducing the intoxicating chemical into the body of the individual.
The term "stimuli exposure history" refers to the status of an individual in terms of repeatedly measured exposures of one or more stimulus to said individual. Stimuli exposure history may for example contain frequency of exposure, intensity of exposure and duration of exposure. Exposure history may optionally be divided into different timeframes. Some non-limiting examples of timeframes may be: Current stimuli exposure which for example may relate to the timeframe of 1-3 days, short term stimuli exposure history which for example may relate to the timeframe of 1-30 days, medium term stimuli exposure history which for example may relate to the timeframe of 10-100 days, and long term stimuli exposure history which for example may relate to the timeframe of 1-6 months to 1-10 years or even longer.
The term "intoxication history" refers to the status of an individual in terms of repeatedly measured content of one or more intoxicating chemical compounds in said individual. Intoxication history may for example contain frequency of intoxication, intensity of intoxication and duration of intoxication. Intoxication history may optionally be divided into different timeframes. Some non-limiting examples of timeframes may be: Current intoxication which for example may relate to the timeframe of 1-3 days, short term intoxication history which for example may relate to the timeframe of 1-30 days, medium term intoxication history which for example may relate to the timeframe of 10-100 days, and long term intoxication history which for example may relate to the timeframe of 1-6 months to 1-10 years or even longer.

The term "clean" refers to the status of an individual as either being completely unaffected by intoxicating chemical compounds or other addictive stimuli, or being exposed to a certain stimulus to a level which is below a predetermined threshold.

The term "central server" refers to a computer which is available through one or more communication protocols such as the internet.

The term "actuator" refers to actuators suitable for providing feedback to the individual. Examples of such actuators include, but are not limited to, mechanical actuators (e.g. similar to the vibration function of a smartphone), optical actuators (e.g. flashing lamp), audio actuators (e.g. playing a sound or a prerecorded voice message), electrical actuators (e.g. transmitting a small but notable current into the individual through electrodes being in contact with the individual), magnetic actuators, and electrical field generating actuators. Other types of actuators include, but are not limited to, showing a picture or a video on a digital screen attached to the device and releasing a chemical agent with a particular smell.

The present invention provides a method to aid in determining if an individual is at risk for a relapse of an addictive behavior. In other words, the invention comprises a method for improved identification of relapse, for example during and after treatment for addictive disorders. The present invention also provides a device suitable for use in the method. The basic principle of the invention is to record the individual's historic exposure to addictive stimuli, for example the use of one or more intoxicating chemical compounds, to evaluate the individuals psychological status through use of a digital questionnaire, and to determine, partly based on the stimuli exposure history, for example in the form of intoxication history, if an individual is at risk for a near-future relapse of addictive behavior. Upon detection of increased risk for relapse, health care providers or other third parties can be alerted so as to support the individual being treated in stopping the relapse.

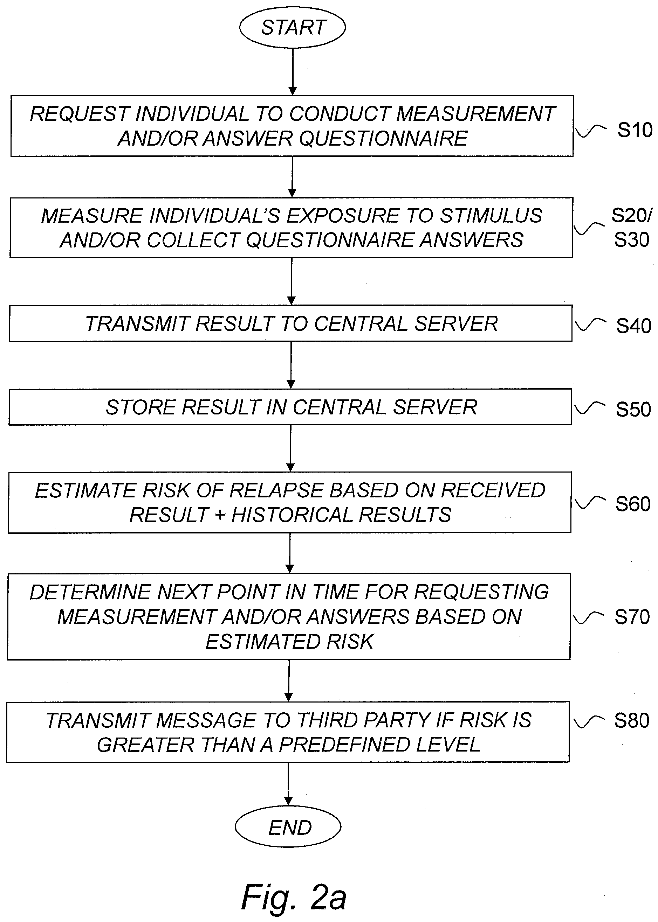

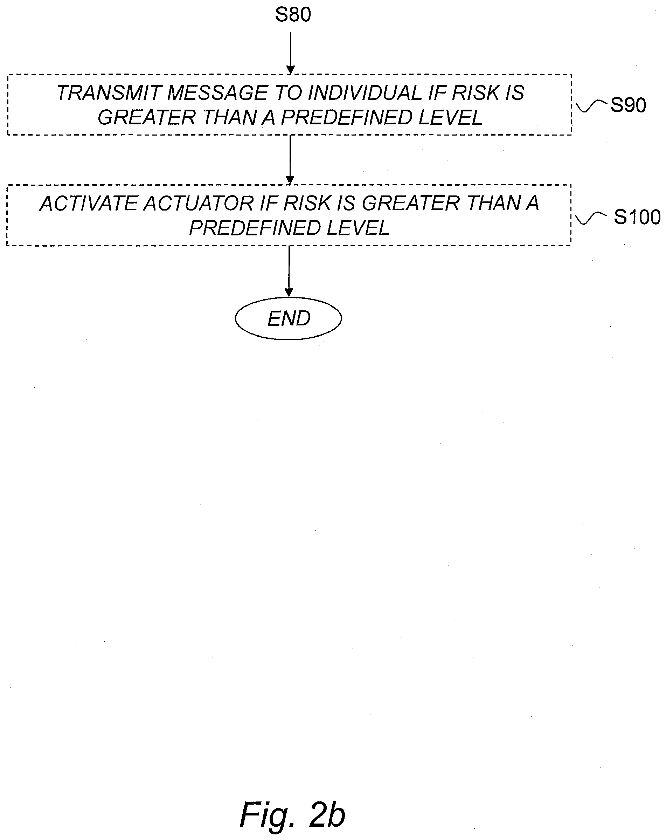

Figure 2 is a schematic flow diagram illustrating an example of a method for estimating a risk of relapse of addictive behavior according to an embodiment. The method comprises performing the following steps at multiple points in time:

- requesting S10 the individual to do at least one of:

  ○ conducting a measurement of the individual's exposure to the stimulus,
  ○ answering a questionnaire provided in a measurement device;

- performing at least one of:

  ○ measuring S20 with the measurement device, the individual's exposure to the stimulus;
  ○ collecting S30, with the measurement device, the individual's questionnaire answers;

- transmitting S40 a result from at least one of the stimulus exposure measurement and the questionnaire answers from the measurement device to a central server unit;
- storing S50 the result in the central server unit;
- upon the central server unit receiving the result, estimating S60 a risk of relapse based on a combination of the received result and historic results;
- determining S70 a next point in time for requesting the individual to do at least one of conducting a measurement of the individual's exposure to the stimulus and answering a questionnaire, based on the estimated risk; and
- if the estimated risk is greater than a predefined level, transmitting S80 a message to a third party.

The method may also be understood as comprising:

- Supplying an individual with a measurement device capable of:

  ○ Measuring the exposure to stimuli
  ○ Presenting questions to a user and collecting answers
  ○ Communicating with a central server unit.

- Said central server unit requesting said individual to use the device at multiple points in time and storing the results in a central server unit.

- Upon the central server unit receiving a result, estimating the risk for relapse of addictive behavior and making the estimate available to a defined third party such as a health care provider or a family member.
- Using the estimated risk for relapse to determine when in time the next measurement is requested.

In some embodiments a request for questionnaire answers is made at the same point in time as a request for a measurement, but it is equally possible to request only a measurement at a certain point in time, or to request only questionnaire answers at a certain point in time. Thus, a request for a measurement and a request for questionnaire answers can be made simultaneously or at different points in time, and with different time intervals or with the same time intervals, if desired.

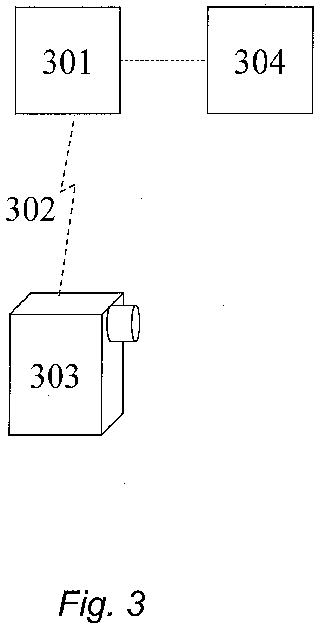

Figure 3 is a schematic diagram illustrating an example of a system configured to estimate a risk of relapse of addictive behavior according to an embodiment. The system comprises a server unit 301 and a measurement device 303, communicating through a wireless connection 302. The central server unit 301 requests an individual to conduct a measurement using the measurement device 303. In an example embodiment the measurement should be conducted within a predetermined time. The measurement device 303 then reports back the result of the measurement to the central server unit 301 using the wireless connection 302, either the measured value or that the measurement was not conducted as requested. Upon the central server unit 301 receiving a result, an estimate of the risk for relapse of addictive behavior is calculated. If the risk for relapse exceeds a predetermined threshold, the central server unit attempts to notify a third party recipient 304, and possibly also the individual (either using an encouraging message or a disturbing action, both for the purpose to disrupt the trend and stop the anticipated relapse). In the latter case the device 303 itself is preferably used. If the risk for relapse is below the predetermined threshold, no action is taken. The estimated risk for relapse is further used to determine the next point in time when a measurement should be requested.

The measurement device 303 shown in figure 3 is configured to enable estimation of a risk of relapse of addictive behavior of an individual, where the addictive behavior is related to exposure to an addictive stimulus. In an embodiment, the measurement device 303 is configured to do the following at multiple points in time:

- perform at least one of:

  ◦ measuring the individual's exposure to the stimulus;
  ◦ providing a questionnaire to the individual and collecting the individual's questionnaire answers;

- and transmit a result from at least one of the stimulus exposure measurement and the questionnaire answers to a central server unit 301 to enable estimation of a risk of relapse.

Thus, the measurement device is capable of measuring the exposure to stimuli, presenting questions to a user and collecting answers, and of communicating with a central server. The measurement device may be one physical unit or may comprise several physical units. One non-limiting example of a measurement device is a two unit device comprising a breathalyzer capable of monitoring alcohol content in an individual's breath and one smart phone capable of presenting questions and communicating with a central server. The two units in this case can be connected using a cable or a short-range communication protocol such as Bluetooth, to mention two non-limiting examples. A non-limiting example of a measurement device comprising one physical unit is a balance capable of measuring the weight of an individual, where the balance is connected to a small computer panel which is capable of presenting questions and collecting answers, and which is capable of communicating with a central server. Still another non-limiting example is a smartphone which is monitoring the user's engagement in on-line gambling. The smartphone is further capable of presenting questions and collecting answers, and which is capable of communicating with a central server. Yet another non-limiting example is the use of wearable sensors (i.e. sensors that are essentially attached to the body of a human, such as a bracelet or sensors embedded into clothes) suitable for detecting a body state which in turn can be linked to stimuli exposure. As an example, one possibility could be to register degree of motion and pulse, where low degree of motion combined with high pulse could e.g. be related to computer gaming but a high degree of motion combined with high pulse excludes computer gaming as a cause for the detected body state. Furthermore, a device may include actuators suitable for providing feedback to the individual. Examples of such actuators include, but are not limited to, mechanical actuators (e.g. similar to the vibration function of a smartphone), optical actuators (e.g. flashing lamp), audio actuators (e.g. playing a sound or a prerecorded voice message), electrical actuators (e.g. transmitting a small but notable current into the individual through electrodes being in contact with the individual), magnetic actuators, and electrical field generating actuators. Other types of actuators include, but are not limited to, showing a picture or a video on a digital screen attached to the device and releasing a chemical agent with a particular smell.

When activating an actuator, it is possible to design the actuator so that it is acting on or near known trigger points on the body. The so-called NADA acupressure points have been used in management of addictive disorders, as discussed for the case of smoking in the report "Effect of self-administered auricular acupressure on smoking cessation--a pilot study" by Leung and co-authors as published in BMC Complement Altern Med. 2012 Feb 28;12:11. doi: 10.1186/1472-6882-12-11. This means, for example, that one or more actuators providing physical pressure on known trigger points can deliver acupressure treatment at points in time where the risk for relapse is elevated. Another non-limiting alternative is to stimulate known trigger points used in acupressure or acupuncture with a small electrical current, so as to deliver treatment at points in time where the risk for relapse is elevated.

The server unit 301 shown in figure 3 is configured to estimate a risk of relapse of addictive behavior of an individual, where the addictive behavior is related to exposure to an addictive stimulus. The server unit 301 is configured to do the following at multiple points in time:

- request an individual to do at least one of:

  ◦ conducting a measurement of the individual's exposure to the stimulus;
  ◦ answering a questionnaire provided in a measurement device 303;

- receive a result from at least one of the stimulus exposure measurement and the questionnaire answers from the measurement device 303;
- store the received result;
- upon receiving the result, estimate a risk of relapse based on a combination of the received result and historic results;
- determine a next point in time for requesting the individual to do at least one of conducting a measurement of the individual's exposure to the stimulus and answering a questionnaire, based on the estimated risk; and
- if the estimated risk is greater than a predefined level, transmit a message to a third party 304.

One important feature of the invention is that the central server unit is requesting the individual to make measurements. This makes it difficult for the individual to hide intentional exposure to stimuli, because the time-points for measurement are out of the individual's control. The request as such can, for example, be communicated through the measurement device as a sound or a lamp being switch on. Another non-limiting alternative is to make the central server unit send a short text message (SMS) to the individual's cell phone. Yet another non-limiting alternative is to make the central server unit call the individual and play a prerecorded phrase which tells the individual that a measurement should be conducted. Still another non-limiting alternative is to make the central server unit send an e-mail to the individual.

The central server attempts to determine the likelihood that the individual is at risk for relapse each time new data is submitted to the central server. The server is then determining (a) if the individual is at elevated risk and (b) a suitable time point for the next request to the individual for presenting a questionnaire. If the central server determines that the individual is, with a high likelihood, at risk for relapse, for example if the risk is above a predefined threshold, a message can be sent to a third party, such as a family member, a health care provider, employer, law enforcement or any other party. This message can be transmitted using the internet or a GSM cell phone network or any other similar network. Additionally, the central server may, if the risk for relapse is exceeding a predetermined level, instruct the device to provide feedback to the individual, so as to attempt to disturb the pattern indicative of relapse and thereby possibly prevent relapse from advancing or even occurring. As an example, the provided feedback may be transmitted as playing a prerecorded message, or sending a short text message (SMS) to the individual's cell phone, with an encouraging content. As another example, the feedback may be of annoying character, such as vibrating the device or playing an annoying alarm.

The measurement device should preferably contain some kind of feature that identifies the individual who is actually using the device. If such a feature is not available, it would be possible for an individual to ask a clean friend to operate the measurement device at times when a measurement is requested. One possible method for identifying the individual who is currently using the measurement device is to let the device take a photo of the operator during the measurement and transfer the photo to the central server together with the obtained result. In case of suspicions regarding an individual attempting to falsify measurements, such photos could be reviewed. Other possible methods for identifying the individual who is operating the measurement device includes, but is not limited to, conducting a retinal or iris scan during measurement, analyzing a voiceprint of the individual or reading a fingerprint during measurement. As an example, a fingerprint reader or other suitable device for identifying the individual could be attached to the measurement device.

The central server unit is typically a computer which comprises a program capable of regularly, or with irregular time intervals, requesting an individual to make measurement, storing measurement results, and estimating if present data is indicative of the individual being clean. The purpose of repeatedly requesting the individual to make measurements is to collect a time-line of stimuli exposure data, such as intoxication data. Such a stimuli exposure history can reveal onset patterns, frequency, duration and intensity of stimuli exposure. Such information is valuable when assessing the likelihood that an individual is clean. For example, if stimuli exposure history indicates that an individual is highly exposed to stimuli approximately once per month with onset in the evening, but the duration is only one day, it may be sufficiently accurate to rely on an evening measurement previous day until the afternoon present day. As another example, if a different individual has a stimuli exposure history which indicates frequent stimuli exposures or intoxications at mild intensity during 3-5 days, a new measurement may be requested multiple times per day.

A key feature of the present invention is the ability to estimate the likelihood of an individual being clean based on historic data. This feature further has an implicit encouragement function: If an individual is succeeding in avoiding exposure to stimuli resulting in a favorable stimuli exposure history, then the individual is implicitly rewarded by a lower frequency of tests. It is possible to use many different underlying methods for determining if the stimuli exposure history, e.g. intoxication history, is indicative of an individual being clean. One non-limiting method is to make a weighted average of the measurement results related to stimuli exposure the recent year, where newer measurements are given higher weight than older. Should an individual be confirmed exposed to stimuli at one point in time, this exposure event will have high impact on the cleanness estimation shortly after, but will gradually reduce in impact as time goes. Another possible method is to extract a short term stimuli exposure history which relates e.g. to the previous month and a long term stimuli exposure history that relates e.g. to approximately the recent year or years, and evaluate if both the short term and the long term history are indicative of cleanness. In the short term assessment, the onset of a relapse can be captured, while as in the long term assessment, the change of the stimuli exposure habit of the individual can be monitored. This makes it possible to determine if a present stimuli exposure event is likely an accident (if the long term history suggests that the individual is staying clean most of the time) or if it likely is part of a pattern suggesting a larger relapse. Such a procedure can be discussed in more detail in the non-limiting context of use of intoxicating chemicals such as alcohol. One possible non-limiting example of a method for combining short term intoxication history with medium term or long term intoxication history for the purpose of determining the cleanness of an individual is the following. First, a short term intoxication (STI) value is calculated. This STI value can be constructed in many different ways, but one possible definition is the following:

$$STI = (\text{(mild intoxication days the recent 30 days)} + 3*\text{(severe intoxication days the recent 30 days)}) / 30$$

The expression "mild intoxication day" could e.g. refer to a day when a measured value of an intoxicating chemical compound above but near the value for confirming intoxication is registered. "Severe intoxication day" could e.g. refer to a day when a measured value of an intoxicating chemical compound is much higher than the value for confirming intoxication is registered. For example, for alcohol, the limit for confirming intoxication could e.g. be "detection limit of the instrument" or it could be "0.2 ‰ Blood alcohol content" and the limit for considering intoxication to be severe could e.g. be "0.5 ‰ Blood alcohol content".

Second, a medium term intoxication (MTI) value can be calculated in a similar manner. One possible definition is the following:

$$MTI = (STI[30]*3 + STI[60]*2 + STI[90]*1) / 6$$

Wherein STI[x] represents the historic STI value at x days before the day of calculating the MTI value, so that STI[1] represents the STI value of yesterday, and STI[2] represents the STI value of the day before yesterday. In plain words, MTI could e.g. be defined as the weighted sum of three historic STI values (in this example one month ago, two months ago and three months ago) where the most recent historic STI value is given higher weight and the oldest STI value is given lowest weight. A long term intoxication (LTI) value can be calculated in still a similar manner, but covering a time period greater than the MTI value (> 3 months in this example). Similar short-term, medium term and long term indicators can be designed also for other stimuli than intoxicating compounds.

Another key feature of the present invention is the ability to investigate the user mood, stamina, and the like through the use of questionnaires. As an optional feature, since the questionnaire is preferably managed through a computer, it is not only the answers to the asked questions (Questionnaire Answers, QA) that may be important, but also how the answers were delivered (Questionnaire Motorics, QM). For example, in a questionnaire that is related to mood, the time spent to answer questions is likely longer at depressed mood than in good mood. Hence, the answers provided to the mood-related questions can be cross checked to the time spent on answering the questions so as to gather both conscious and motoric information on the same subject for the purpose of producing a more reliable estimate of the true mood of the individual. In a similar manner, if a questionnaire is related to alcohol consumption, the speed and motoric precision will probably be reduced also at low level intoxication. This means that if a multiple-choice question is presented, and time to answer combined with the distance between where on the screen the individual indicates the answer as compared to the position of the indicators for the multiple choice answers will reveal the speed and motoric precision of the individual. When combining the conscious and motoric information on the same subject for the purpose of producing a more reliable estimate of the true condition of the individual. It is beneficial to gather baseline information about each individual to make possible comparisons of current Questionnaire Answers (QA) and current

Questionnaire Motorics (QM) so that deviations from the normal state of an individual can be evaluated.

By combining intoxication measures of different time-frames like e.g. STI, MTI, LTI, QA, QM and/or other data a reliable measure of the likelihood of being at risk for relapse can be created. Other data includes, but is not limited to, time of day, day of week, result of the previous measurement, elapsed time since the previous measurement, number of measurements that have been missed in the recent past, and similar.

In some cases it may, for pedagogic reasons in relation an individual suffering from addictive disorder, be favorable to present an intoxication index as a sobriety index. A sobriety index is always negatively correlated with an intoxication index.

It is also possible to supplement the method with a series of other functions, including but not limited to, monitoring that the measurement device (and hence presumably the individual) has been in close proximity to a predefined geographic position at a predetermined time point (such as meetings with a therapist or meetings with support groups) through use of GPS (global positioning system).

The questionnaire that may for example be embedded in a hand held device, such as a smartphone, and can be configured to at different frequencies (e.g. at least once per week, more preferably once or twice per day, and in extreme cases once per hour during daytime) ask an individual questions that are related to stimuli exposure or that reveal increased risk for the individual exposing himself or herself. Suitable questionnaires may be selected from those available and validated, such as questionnaires to monitor addiction (where AUDIT is one non-limiting example) and psychological status (where MADRS is one non-limiting example). Questionnaires may also cover areas like daily mood and exercise of routines (work, school, therapy), motivation, eating habits, sleeping habits, engagement and self-efficacy, to mention a few non-limiting examples. These data can in the same way as measurements of stimuli exposure be compressed and compiled into short, medium and long term risk factors (SRF, MRF, LRF) useful for estimation of the risk of stimuli exposure. For example, if the motivation level to be clean is low and the daily mood is poor the risk of self-inflicted stimuli exposure is usually higher and therefore measurements and questionnaires should be performed more frequently.

The actual measurement results related to intoxication can potentially be combined with a range of additional data (some of which are exemplified above, and potentially compressed into variables like SRF, MRF, and LRF), in the process of determining if an individual is likely to be clean or not.

The present invention provides a non-transitory computer readable medium comprising instructions for causing a computer to perform particular steps of the above-described method, such as the estimation of if an individual is likely to be clean which are typically conducted in the central server computer. Furthermore, the measurement device may contain a non-transitory computer readable medium comprising instructions for causing a computer to perform parts of the procedures related to the direct or indirect measurement of stimuli exposure, such as measuring the quantity of a chemical substance, measuring the motoric reaction pattern of an individual, measuring a physical property such as weight of an individual, to mention a few non-limiting examples.

An example of a suitable type of measurement device comprises an apparatus capable of measuring breath alcohol content by analyzing the exhaled air of an individual, where breath alcohol level is a surrogate for blood alcohol level. Such devices are commonly known as breathalyzers. One possibility is use a breathalyzer of type Kontigo tripleA. This device measures the Breath Alcohol (BrAC) Level of an individual using a sampling pump and a fuel cell which converts alcohol content to an electric signal, said signal being dependent on the alcohol concentration. The breath sample is taken when the individual has delivered more than 1.2 liters of air directly from his/her lungs. The electric signal is processed and converted to digital form in a microcontroller and is then compared to calibration values to allow transformation to a corresponding BrAC-level in mg/L. When the BrAC-level has been calculated it can then be transmitted to a connected host smart phone which in turn can deliver the value to a central server unit. Before, during and after the measurement the actual state is visualized to the user with light emitting diode indications in various colors. The Kontigo tripleA device is further equipped with a camera which is depicting the individual during the measurement, so as to provide evidence that it is a particular individual operating the device. This above described example of a measurement device hence comprises two parts: one part that contains a breath analyzer and one other part that is a regular smart phone capable of presenting questions and collecting answers, and communicating with both the breath analyzer and central server units. By dividing the measurement device into two parts where one, the smart phone, is a regular existing product the cost for the measurement device is reduced.

It is important to mention that for a measurement device intended to quantify an intoxicating chemical compound, it is not necessary to measure the exact intoxicating chemical compound. It is in some cases possible to measure a metabolite or a catabolite that is related to the intoxicating compound. The measurement of ketone which is related to alcohol consumption is one such indirect measurement that is capable of determining if an individual has consumed alcohol. Furthermore, measurement devices that conduct the measurement of one or more intoxicating chemical compounds are typically using a non-invasive sample specimen. Examples of non-invasive sample specimens include, but are not limited to, analysis of urine, feces, sweat, saliva, and exhaled air.

The present invention makes it possible to use stimuli exposure history to determine if a health care provider should follow up an individual with known addiction behavior. It is well known that in the care of alcoholics, drug addicts, and individuals with similar diseases or disorders, there is a high frequency of relapse after treatment. As a particular example, in the care of alcoholics it has been reported that even after treatment, the average short-term abstinence rates is only approximately 40% as discussed in the report "Rates and predictors of relapse after natural and treated remission from alcohol use disorders" by RH Moos and BS Moos as published in Addiction. Feb 2006; 101(2): 212-222. This means that the society has a lot to benefit from improved care of alcoholics after the actual treatment has taken place. The present invention can be applied as a monitoring tool where the intoxication history is captured and stored, and in cases where an individual is determined to be intoxicated a message can be sent to the health care provider. The present invention is thus capturing relapse of disease in an early stage, which allows health care providers, family members, and other related individuals to become informed so as to attempt to interrupt the relapse. The present invention may, when used as a monitoring tool, be supplemented with a series of other functions, such as support e.g. in the form of encouraging text messages or e-mails, possibly synchronized with times or geographic locations where risk for relapse is high (for example Friday evening in a restaurant area).

Addictive behavior need not be linked to a chemical compound. An individual may become addicted to e.g. gambling, and with the availability of a multitude of on-line gambling web-sites it may be difficult for an individual to stay clean from gambling. Aspects of pathological gambling is discussed in the report "Retrospective and Prospective Reports of Precipitants to Relapse in Pathological Gambling." by Hodgins, David C. and el-Guebaly, Nady as published in Journal of Consulting and Clinical Psychology, Vol 72(1), Feb 2004, 72-80. http://dx.doi.org/10.1037/0022-006X.72.1.72. Another example is pathological relationships to food, leading to an un-healthy situation (such as anorexia or obesity).

In a particular example embodiment, the method comprises the steps of

- Estimating said individual's exposure to said stimuli by use of a measurement device.
- Requesting said individual to complete a questionnaire in said measurement device.
- Transmitting the results from said stimuli exposure estimate and the questionnaire answers to a central server.
- Upon said central server receiving results from said measurement device, combining the received results with historic results and other data to determine the risk for relapse.
- If said risk for relapse is greater than a predefined level, notifying a third party

WHEREIN

- Said the step of estimating exposure to stimuli is conducted at least once per week.
- The answers to the questions in said questionnaire comprises (a) the individual's answer to the question and (b) at least one measurement of how said answer was delivered.
- Said determination of risk for relapse is made based on a combination of

  ◦ measured stimuli exposure during at least the recent month
  ◦ questionnaire results during at least the recent month
  ◦ other information

In the above-mentioned method, the third party may be one or more of:

- A health care provider
- A family member or close relative
- An employer
- A law enforcement organization
- A governmental institution

According to one embodiment of the present invention, the determination of risk for relapse is made based on a combination comprising values related to (a) short-term stimuli exposure history covering the recent 3 to 5 weeks, (b) medium-term stimuli exposure history covering recent 3 to 5 months, and (c) other information.

According to another embodiment of the present invention, the determination of risk for relapse is made based on a combination comprising values related to said individual taking part in a support program or treatment program related to addictive exposure to stimuli.

According to yet another embodiment of the present invention, the method is related to the stimuli alcohol. In such a case, the measurement device may comprise a capability of measuring breath alcohol content.

According to still another embodiment of the present invention, the method is related to the stimuli gambling or gaming.

According to yet another embodiment of the present invention, the method is related to the stimuli food.

According to still another embodiment said at least one measurement of how said answer was delivered may comprise measuring the time from displaying a questions to answering the question; or measuring the distance from the center of a button representing the selected answer to the position that was actually pressed by said individual while answering the question.

Example 1

In the present example, the ability for a measurement device to capture alcohol consumption through use of a small and simple questionnaire was tested. A questionnaire was designed to have three questions.

Question one was "Are you capable of walking 5 m on a straight line painted on the floor?" Three possible answers were made available: Yes, No, Maybe.

Question two was "Please estimate your current blood alcohol level". The answer was provided through setting a marker on a continuous scale.

Question three was "Are you capable of driving a vehicle?" Three possible answers were made available: Yes, No, Maybe.

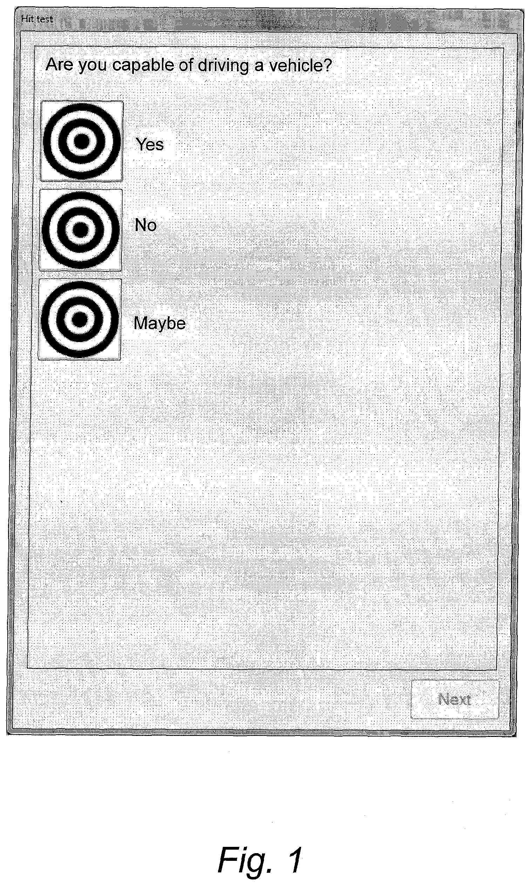

The multiple choice questions (1 and 3) had large buttons (approximately 2*2 cm) with concentric circles, as shown in figure 1. The questionnaire was programmed as a standalone application and was run on a tablet computer equipped with a touchscreen, so that the individual being tested used his or her finger or a pen suitable for touchscreens to indicate answers. When the questionnaire was run, not only the answers were recorded, but also (1) time to answering and for multiple choice questions (2) the distance from the center of the button representing the selected answer to the position that was actually pressed by the individual.

The questionnaire was tested using a male (66 years old, 167 cm tall, weight 62 kg, Swedish citizen) who participated voluntary. The tester was instructed to run the questionnaire approximately every 15 minutes. After each questionnaire, the tester was asked to measure the alcohol level by using a breathalyzer of model Kontigo TripleA. During the first hour of the test, no alcohol was consumed.

Starting at one hour, the tester was instructed to drink approximately 1 unit of alcohol (12 cl wine of 13% alcohol level) per 20 minutes until the breath alcohol level exceeded 0.6 mg/L (which approximately corresponds to 0.12% blood alcohol). During this experiment, the tester was not aware of the fact that the questionnaire was collecting motoric information.

Results obtained for question 1 were omitted, because the tester was not always in focus for answering questions when starting the questionnaire, in particular after having consumed some alcohol. Results for question 2 were also omitted because the technical solution for the continuous scale was not sufficiently user-friendly. Results for question 3 are found in Table 1. At question 3, the tester had obtained focus on the questionnaire and the question as such was technically simple to answer. Which of the three possible answers the test subject responded at different times is ignored.

Table 1: Results for the question: "Are you capable of driving a vehicle?"

| Time-point | Alcohol consumption | Alcohol level in breath | Distance from center of button to position pressed |
|---|---|---|---|
| | cl wine (13%) | mg/L | pixels |
| 18:12 | 0 | 0 | 9.43 |
| 18:25 | 0 | 0 | 6.40 |
| 18:32 | 0 | 0 | 8.54 |
| 18:46 | 12 | 0 | 23.43 |
| 18:56 | 24 | 0.049 | 3.61 |
| 19:16 | 36 | 0.101 | 17.46 |
| 19:31 | 48 | 0.279 | 32.39 |
| 19:50 | 60 | 0.283 | 4.12 |
| 20:09 | 60 | 0.373 | 21.95 |
| 20:27 | 72 | 0.54 | 19.72 |
| 20:45 | 72 | 0.602 | 23.26 |
| 21:07 | 72 | 0.628 | 19.42 |

In the beginning of the experiment, when the tester was unaffected by alcohol, the typical distance from button center was about 10 pixels. After having consumed alcohol, the typical distance from button center increased to about 20 pixels. This indicates that the precision of pressing a large button shaped to indicate a center position decreases when the tester is under the influence of alcohol. It would therefore be possible to combine the motoric information with the actual answers to questions to better determine the status of an individual.

Example 2

This example shows that it is possible to estimate if an individual is at risk being intoxicated. Twelve individuals known to have an adverse drinking behavior were provided with a portable blood alcohol measurement device (Kontigo tripleA) which is communicating with a central server unit for receiving information about when next measurement is scheduled and for storage of measurement results. At the time a of blood alcohol measurement, a brief questionnaire with questions related to mood and well-being was shown to the individual and questionnaire results are stored in the central server unit. For each individual, a sobriety index (SI) for monitoring misuse of alcohol of an individual was composed of the actual measured blood alcohol values (requested three times per day) combined with recent missed measurements. Unwanted results (elevated blood alcohol level or missed measurement) were given additional penalty if occurring evening or morning, and if occurring consecutively. In schematic equation form, SI was calculated based on "raw sobriety index" (rawSI) according to

$$rawSI = 100 - (A + B*[timepoint\ penalty]) * [BAC] - C*[missedBAC] - D * [consecutive\ penalty]$$

where A, B, C, and D are coefficients or weights, [timepoint penalty] is 1 if the most recent measurement of blood alcohol was evening or morning or a Friday and 0 otherwise, [BAC] is the most recent blood alcohol level measurement (expressed in g/L), [missedBAC] is 1 if the most recent request for measurement of blood alcohol was not conducted within the stipulated time-frame and 0 otherwise, and [consecutive penalty] is 1 if both the most recent blood alcohol measurement and the second most recent blood alcohol measurement both had either elevated blood alcohol level or were not conducted within the stipulated time-frame (and 0 otherwise). The sum of the coefficients (A + B) is approximately 40-60 divided by the blood alcohol legal limit for driving a car. The coefficient C is approximately 40-60, and the coefficient D is approximately 100-C. Sobriety index SI is calculated as the average of rawSI obtained three times per day during the recent 7 days.

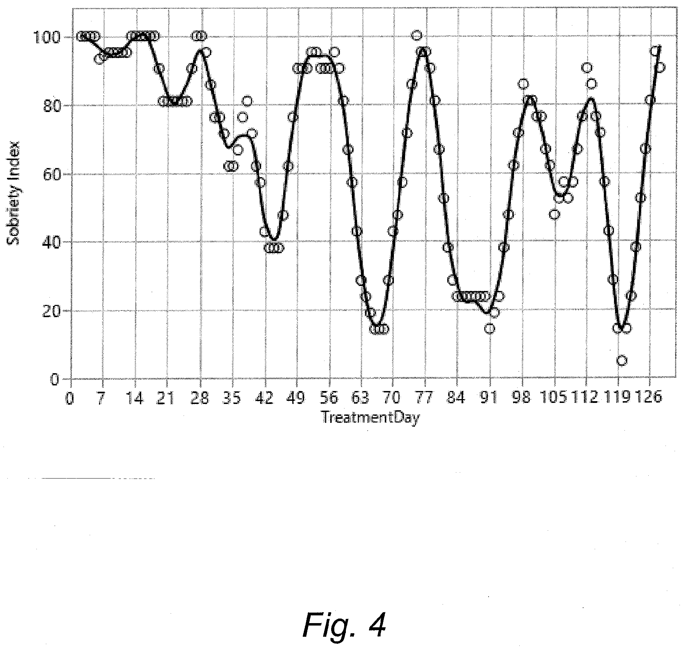

Figure 4 shows the sobriety index plotted versus time during 126 days for an individual who was in treatment for alcohol abuse. This individual was in confirmed relapse approximately every 14 days, which was occurring at the same time as sobriety index was below 60.

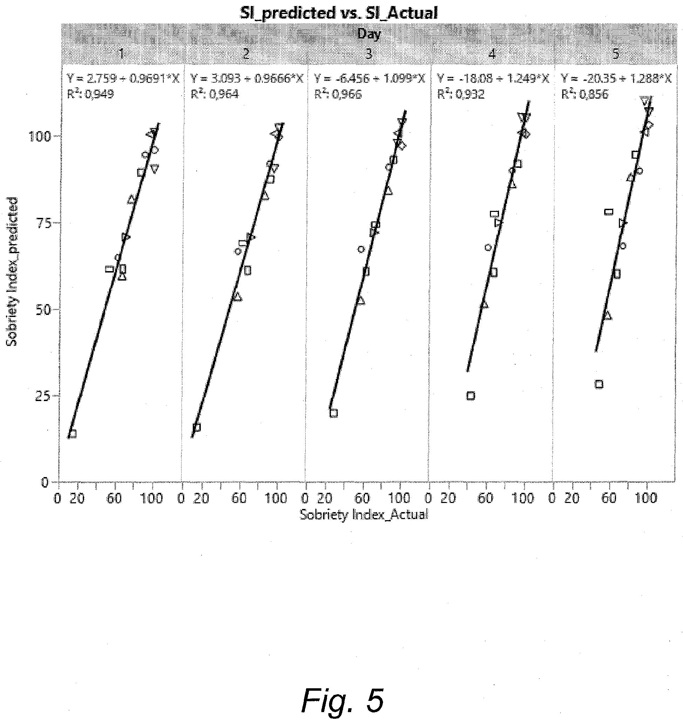

When applying a time-series model to the series of SI, prediction of SI for future time points can be conducted. Figure 5 shows the prediction of SI plotted versus actual SI for 12 individuals. When fitting a straight line to the predicted versus observed scatter plot, the intercept is close to zero (2.7 to 6.4), the coefficient close to one (0.97-1.01) and R-square is 0,95-0.96 when predicting SI the first, second and third day into future. The model becomes less accurate day 4-5.

This example illustrates, using real data collected from a multitude of individuals during more than 100 days, that it is possible to calculate a sobriety index which is suitable for prediction of near-future sobriety.

Example 3

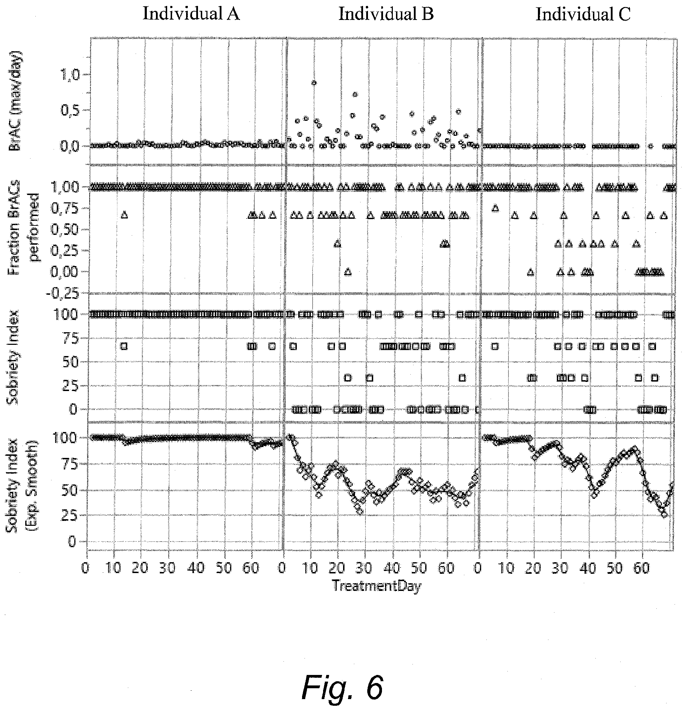

This example shows that it is possible to use SI for estimating if an individual is at risk being intoxicated. Data from three individuals being part of the same clinical study as described in Example 2 above are shown in Figure 6. The three individuals were, in addition to being part of the monitoring program, also monitored using biomarkers indicative of alcohol consumption, including blood biomarkers Phosphatidylethanol (PEth) and Carbohydrate-Deficient Transferrin (CDT). Urine biomarkers ethyl glucuronide (EtG) and ethyl sulfate (EtS) can also be used for monitoring short term consumption. Since PEth is eliminated from the body with approximately 4-5 days half-life, it is a suitable biomarker for confirming alcohol consumption recent week. Use of PEth, CDT, EtG and EtS has been discussed in "Monitoring of the alcohol biomarkers PEth, CDT and EtG/EtS in an outpatient treatment setting." as published by Helander and co-authors in Alcohol Alcohol. 2012 Sep-Oct;47(5):552-7. doi: 10.1093/alcalc/ags065.

In figure 6, the top row of graphs corresponds to the maximum breath alcohol registered during one day, the second row of graphs corresponds to the compliance of measurement, i.e. the number of reported measurements divided by the number of requested measurements. The third row of graphs corresponds to the current sobriety index and the bottom row of graphs corresponds to a smoothed sobriety index (using data from 7 consecutive days to estimate a smoothened sobriety index at each point in time).

Individual A (left column of graphs) is an individual who is not drinking alcohol in any measurable quantities. The breath alcohol level is consistently near zero throughout the 70 consecutive days of monitoring (top-left graph). The compliance to measuring alcohol of individual A is very high (second row, left column graph). Accordingly, the sobriety index is high, indicating that this individual is likely sober which was also confirmed by the biomarkers.

Individual B (middle column) is an individual who is drinking heavily from time to time while having a fair compliance to measuring breath alcohol. With few exceptions, at the most one measurement per day was missed. The sobriety index is low during drinking periods and recovers to higher values in-between. For individual B, drinking was confirmed using biomarkers.

Individual C (right column) is an individual who drinks heavily (as confirmed by biomarkers), but who intentionally avoids measuring breath alcohol as requested when intoxicated. The sobriety index is reduced during periods of missed measurements, meaning that intoxication was detected through absence of results rather than presence of confirmed alcohol consumption.

This example illustrates, using real data collected from three individuals, that it is possible to calculate a sobriety index also in cases where measurement compliance is low.

Example 4

This example shows that it is possible to estimate if an individual is at risk being intoxicated. Within the study as described in Example 2, a subset of the individuals were using measurement devices that were presenting a questionnaire related to mood at least daily (and transferring results to the central server). For these individuals, a sobriety index (SI) for monitoring misuse of alcohol of an individual was composed of the actual measured blood alcohol values (requested three times per day) combined with recent missed measurements and combined with a mood parameter. Unwanted results (elevated blood alcohol level or missed measurement) were given additional penalty if occurring evening or morning, and if occurring consecutively. In schematic equation form, SI was calculated based on "raw mood adjusted sobriety index" (MASi) according to MASi = 100 - (A + B*[timepoint penalty]) * [BAC] - C*[missedBAC] - D * [consecutive penalty] - E [mood change] where A, B, C, D, and E are coefficients or weights, [timepoint penalty] is 1 if the most recent measurement of blood alcohol was evening or morning or a Friday and 0 otherwise, [BAC] is the most recent blood alcohol level measurement (expressed in g/L), [missedBAC] is 1 if the most recent request for measurement of blood alcohol was not conducted within the stipulated time-frame and 0 otherwise, [consecutive penalty] is 1 if both the most recent blood alcohol measurement and the second most recent blood alcohol measurement both had either elevated blood alcohol level or were not conducted within the stipulated time-frame (and 0 otherwise), and [mood change] is > 1 if the most recent questionnaire results indicate that mood has changed as compared to the recent 7 days. The sum of the coefficients (A + B) is approximately 40-60 divided by the blood alcohol legal limit for driving a car. The coefficient C is approximately 40-60, the coefficient D is approximately 90-C and the coefficient E is approximately 10.

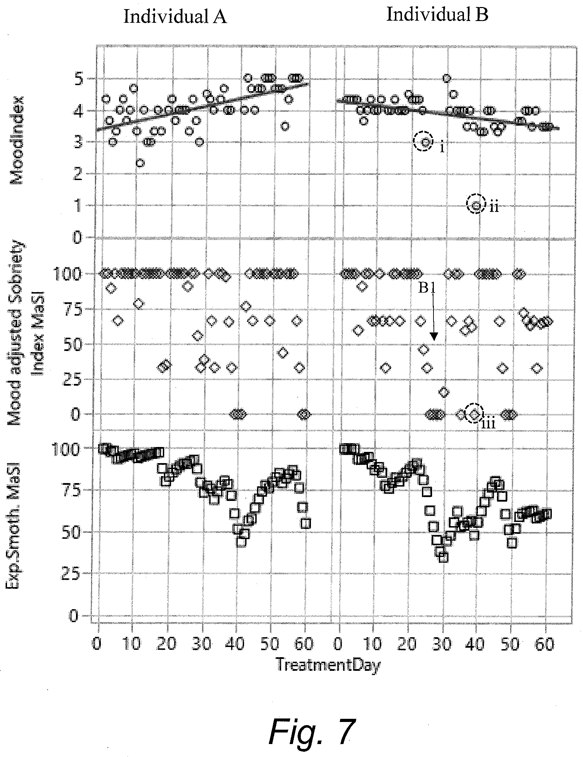

In figure 7, the top row of graphs corresponds to Mood index which is the estimation of mood based on questionnaire results, the middle row corresponds to MASi and the lower row corresponds to Exponentially smoothened MASi data, which is one possible way to construct a Sobriety index SI based on the raw MASi data. The left column refers to data from one individual (A) and the right column to another individual (B). The x-axis is in all cases treatment day.

MoodIndex (MI) vs. TreatmentDay (upper row of graphs) shows two deviating patterns for individual A and individual B. For individual A, MI increases with time. Individual A suffers from a confirmed relapse around treatment day 40 and reports consistently increasing MI. This individual may be at risk for relapse when mood is better than average, but in this example MI was not capable of identifying the onset of a relapse. For individual B, MI is decreasing over time. The relapse at approximately day 30 starts with an indication of decreased mood (i) and the arrow B1 shows the start of a 4 day long confirmed relapse. The relapse at day 30 was identified partly due to use of MI. For individual B there is a strong dip in mood around day 40 (ii) which does not influence the mood adjusted sobriety index (middle row of graphs) because it is already zero (iii) due to a positive measurement of breath alcohol. The MI adjusted sobriety index (low, MaSI shown as exponentially smoothed data) opens up the possibility to classify patients into groups which might take the relapse during high (e.g. individual A) and/or low (individual B) and/or independent of mood state(s) (B). This means that the deviation (in any direction) of current mood from recent historic mood may be a strong indicator of elevated risk for relapse, which in turn constitutes an elevated risk for being intoxicated.

Example 5

This example shows that it is possible to use SI for estimating if an individual is at risk being intoxicated. Data from the individuals being part of the clinical study as described in Example 2 above was subjected to time-series analysis using a modified Holt-Winters additive seasonal method.

$$\hat{Y}_{t+h|t} = l_t + hb_t + s_{t-m+h_m^+}$$

$$l_t = \alpha(Y_t - s_{t-m}) + (1 - \alpha)(l_{t-1} + b_{t-1})$$

$$b_t = \beta(l_t - l_{t-1}) + (1 - \beta)b_{t-1}$$

$$s_t = \gamma(Y_t - l_{t-1} - b_{t-1}) + (1 - \gamma)s_{t-m}$$

$$h_m^+ = \lfloor (h - 1) \bmod m \rfloor + 1$$

Where $\hat{Y}$ is the estimated SI, l is the magnitude quantity, b is the trend quantity, s is the seasonality quantity, and h is a periodic (in this case week-day) correction factor (m=7). The coefficients $\alpha$, $\beta$, and y have values between 0 and 1 and are estimated in an individualized manner (i.e. each individual has unique values of $\alpha$, $\beta$, and $\gamma$). The coefficients of Holt-Winters methods are estimated in an incremental manner: The initial guess of the magnitude quantity is typically the first measured quantity (i.e. SI at first day in this particular case; I = SI(at day 1)) and the initial guess of the trend quantity is often the difference between the first two measured quantities (i.e. SI(day 2) - SI(day 1)). Holt-Winters methods is typically made to operate in an adaptive manner and coefficients can be recalculated at any point in time so that all recent data is fully included in the time series prediction. The Holt-Winters additive seasonal method has been discussed in the report "Comparison of Statistical Models for Analyzing Wheat Yield Time Series." by Michel and Makowski as published in PLoS ONE 8(10): e78615. doi:10.1371/journal.pone.0078615.

SI was defined as discussed in previous examples and was scaled so that a completely clean individual had SI = 100 and a completely intoxicated individual had SI = 0.

When applying the Holt-Winters additive seasonal method to one of the individuals for which 187 days of measurement data existed, the Holt-Winters additive seasonal method could predict SI of tomorrow to the level that in 87% of the cases the predicted SI was deviating less than 10 units from the measured SI (which ranges from 0 to 100). For many of the evaluated individuals, the prediction accuracy was greater than 70%. Some individuals did however have spurious drinking patterns for which the Holt-Winters additive seasonal method had lower performance indicating that either longer follow-up time is required for the model to adapt to the behavioral pattern of such individuals, or that a different model for estimating SI is required for such individuals.

This example shows that for a subset of the evaluated individuals, SI can be estimated and predicted with high accuracy using Holt-Winters additive seasonal method.

The embodiments described above are merely given as examples, and it should be understood that the proposed technology is not limited thereto. It will be understood by those skilled in the art that various changes, combinations and modifications may be made without departing from the scope of the invention as set forth in the claims appended hereto. In particular, different part solutions in the different embodiments can be combined in other configurations, where technically possible.

**Claims**

1. Method for estimating a risk of relapse of addictive behavior of an individual, said addictive behavior being related to exposure to an addictive stimulus, said method comprising:
   At multiple points in time, repeatedly

   • requesting (S10), by a central server unit (301), said individual to do at least one of:

     ○ conducting a measurement of said individual's exposure to said stimulus;
     ○ answering a questionnaire provided in a measurement device (303);

   • performing at least one of:

     ○ measuring (S20), with said measurement device (303), said individual's exposure to said stimulus;
     ○ collecting (S30), with said measurement device (303), said individual's questionnaire answers;

   • transmitting (S40) a result from said stimulus exposure measurement, if performed, and said questionnaire answers, if collected, from said measurement device (303) to said central server unit (301);
   • storing (S50) said result in said central server unit (301);
   • upon said central server unit (301) receiving said result, said central server unit (301) estimating (S60) a risk of relapse based on a combination of the received result and historic results, and at least one of the following:

- time of day when measuring said individual's exposure to said stimulus;
- day of week when measuring said individual's exposure to said stimulus;
- result of previous measurement of said individual's exposure to said stimulus;
- elapsed time since previous measurement of said individual's exposure to said stimulus;
- number of historic measurements of said individual's exposure to said stimulus that have been missed;

• determining (S70), by said central server unit (301), a next point in time for requesting said individual to do at least one of conducting a measurement of said individual's exposure to said stimulus and answering a questionnaire, based on said estimated risk, wherein the next point in time is determined so as to collect a time-line of stimuli exposure data that reveals onset patterns, frequency, duration and intensity of stimuli exposure; and
• if said estimated risk is greater than a predefined level, transmitting (S80), from said central server unit (301), a message to a third party (304), wherein said third party is at least one of:

- a health care provider;
- a family member or close relative;
- an employer;
- a law enforcement organization; and
- a governmental institution.

2. Method of claim 1, further comprising, if said estimated risk is greater than a predefined level, providing feedback to said individual by performing transmitting (S90) a message to said individual.

3. Method of claim 1 or 2, further comprising identifying said individual during measurement of said individual's exposure to said stimulus.

4. Method of any preceding claim, wherein said estimating a risk of relapse is based on a combination of:

• historic results from at least one of said stimulus exposure measurements and said questionnaire answers covering the recent 1-30 days, and
• historic results from at least one of said stimulus exposure measurements and said questionnaire answers covering the recent 10-100 days.

5. Method of any preceding claim, wherein said estimating a risk of relapse is further based on at least one of:

• said individual's participation in a support program or treatment program related to addictive behavior;
• short, medium and long term risk factors including at least one of daily mood, exercise of routines such as work routines, school routines and therapy routines, motivation, eating habits, sleeping habits, engagement and self-efficacy as assessed in said questionnaire; and
• motoric information on how questionnaire answers were delivered.

6. Method of any of the claims 1-5, wherein said stimulus is alcohol.

7. Method of any of the claims 1-5, wherein said stimulus is gambling or gaming.

8. Method of any of the claims 1-5, wherein said stimulus is food.

9. Server unit (301) configured to estimate a risk of relapse of addictive behavior of an individual, said addictive behavior being related to exposure to an addictive stimulus, wherein the server unit (301) is configured to:
At multiple points in time, repeatedly

• request an individual to do at least one of:

- conducting a measurement of said individual's exposure to said stimulus;
- answering a questionnaire provided in a measurement device (303);

• receive a result from said stimulus exposure measurement, if performed, and said questionnaire answers, if collected, from said measurement device (303);
• store said received result;
• upon receiving said result, estimate a risk of relapse based on a combination of the received result and historic results, and at least one of the following:

  ◦ time of day when measuring said individual's exposure to said stimulus;
  ◦ day of week when measuring said individual's exposure to said stimulus;
  ◦ result of previous measurement of said individual's exposure to said stimulus;
  ◦ elapsed time since previous measurement of said individual's exposure to said stimulus;
  ◦ number of historic measurements of said individual's exposure to said stimulus that have been missed;

• determine a next point in time for requesting said individual to do at least one of conducting a measurement of said individual's exposure to said stimulus and answering a questionnaire, based on said estimated risk, wherein the next point in time is determined so as to collect a time-line of stimuli exposure data that reveals onset patterns, frequency, duration and intensity of stimuli exposure; and
• if said estimated risk is greater than a predefined level, transmit a message to a third party (304), wherein said third party is at least one of:

  ◦ a health care provider;
  ◦ a family member or close relative;
  ◦ an employer;
  ◦ a law enforcement organization; and
  ◦ a governmental institution.

10. Server unit (301) of claim 9, wherein the server unit (301) is configured to estimate a risk of relapse based on a combination of:

  • historic results from at least one of said stimulus exposure measurements and said questionnaire answers covering the recent 1-30 days, and
  • historic results from at least one of said stimulus exposure measurements and said questionnaire answers covering the recent 10-100 days.

11. Server unit (301) of claim 9 or 10, wherein the server unit (301) is further configured to estimate a risk of relapse based on at least one of:

  • said individual's participation in a support program or treatment program related to addictive behavior;
  • short, medium, and long term risk factors including at least one of daily mood, exercise of routines such as work routines, school routines and therapy routines, motivation, eating habits, sleeping habits, engagement and self-efficacy as assessed in said questionnaire; and
  • motoric information on how questionnaire answers were delivered.

12. System configured to estimate a risk of relapse of addictive behavior of an individual, said addictive behavior being related to exposure to an addictive stimulus, wherein the system comprises a server unit (301) of any of the claims 9 to 11, and a measurement device (303) configured to:

  At multiple points in time,

    • perform at least one of:

      ◦ measuring said individual's exposure to said stimulus;
      ◦ providing a questionnaire to said individual and collecting said individual's questionnaire answers;

    • transmit a result from said stimulus exposure measurement, if performed, and said questionnaire answers, if collected, to a central server unit (301) to enable estimation of a risk of relapse,

  and wherein the measurement device (303) is further configured to enable identification of said individual during measurement of said individual's exposure to said stimulus.

13. System of claim 12, wherein the measurement device (303) comprises at least one of the following:

- a breathalyzer configured to measure an alcohol content in said individual's breath;
- a motion sensor configured to measure motion of said individual;
- a pulse sensor configured to measure pulse of said individual;
- a balance configured to measure a weight of said individual.
- a smart mobile phone configured to at least one of the following:

    ◦ present questions, collect answers and communicate with a central server unit (301);
    ◦ monitor said individual's engagement in online gambling or gaming.

14. Computer-program product comprising a computer-readable medium having stored thereon a computer program comprising instructions, which when executed by at least one processor, cause the at least one processor to perform steps of the method of any of the claims 1 to 8.


**Patentansprüche**

1. Verfahren zur Abschätzung des Rückfallrisikos einer Person in ein Suchtverhalten, das mit der Exposition gegenüber einem Suchtreiz in Zusammenhang steht, wobei das Verfahren Folgendes umfasst:
Zu mehreren Zeitpunkten wiederholt

- Aufforderung (S10) der Person durch eine zentrale Servereinheit (301) zur Durchführung mindestens eines der folgenden Verfahren:

    ◦ Durchführung einer Messung der Exposition der Person gegenüber dem Reiz;
    ◦ Beantwortung eines in einem Messgerät (303) bereitgestellten Fragebogens;

- Durchführung mindestens eines der folgenden Verfahren:

    ◦ Messung (S20) der Exposition der Person gegenüber dem Reiz mit dem Messgerät (303);
    ◦ Erfassung (S30) der Fragebogenantworten der Person mit dem Messgerät (303);

- Übermittlung (S40) eines Ergebnisses der Reizexpositionsmessung (sofern durchgeführt) und der Fragebogenantworten (sofern erfasst) vom Messgerät (303) an die zentrale Servereinheit (301);
- Speicherung (S50) des Ergebnisses in der zentralen Servereinheit (301);
- Nach Erhalt des Ergebnisses durch die zentrale Servereinheit (301) schätzt (S60) die zentrale Servereinheit (301) das Rückfallrisiko auf Grundlage einer Kombination aus dem empfangenen Ergebnis und historischen Ergebnissen sowie mindestens einem der folgenden Parameter:

    ◦ Tageszeit der Messung der Reizexposition der Person;
    ◦ Wochentag der Messung der Reizexposition der Person;
    ◦ Ergebnis der vorherigen Messung der Reizexposition der Person;
    ◦ Verstrichene Zeit seit der vorherigen Messung der Reizexposition der Person;
    ◦ Anzahl der verpassten Messungen der Reizexposition der Person;

- Festlegen (S70) eines nächsten Zeitpunkts durch die zentrale Servereinheit (301) für die Aufforderung an die Person, basierend auf dem geschätzten Risiko mindestens eine Messung ihrer Reizexposition durchzuführen und einen Fragebogen zu beantworten, wobei der nächste Zeitpunkt so festgelegt wird, dass ein Zeitverlauf der Reizexpositionsdaten erfasst wird, der Beginn, Häufigkeit, Dauer und Intensität der Reizexposition aufzeigt; und
- Übersteigt das geschätzte Risiko einen vordefinierten Wert, wird von der zentralen Servereinheit (301) eine Nachricht an einen Dritten (304) gesendet (S80); wobei dieser Dritte mindestens einer der folgenden ist:

    ◦ ein Gesundheitsdienstleister;
    ◦ ein Familienmitglied oder naher Verwandter;
    ◦ ein Arbeitgeber;
    ◦ eine Strafverfolgungsbehörde; und
    ◦ eine staatliche Institution.

2. Verfahren nach Anspruch 1, das ferner umfasst, dass, wenn das geschätzte Risiko einen vordefinierten Wert überschreitet, der betreffenden Person durch Senden (S90) einer Nachricht eine Rückmeldung gegeben wird.

3. Verfahren nach Anspruch 1 oder 2, das ferner umfasst, die betreffende Person während der Messung ihrer Reizexposition zu identifizieren.

4. Verfahren nach einem der vorhergehenden Ansprüche, wobei die Abschätzung des Rückfallrisikos auf einer Kombination folgender Faktoren basiert:

   • historischen Ergebnissen aus mindestens einer der Reizexpositionsmessungen und den Fragebogenantworten der letzten 1-30 Tage, und
   • historischen Ergebnissen aus mindestens einer der Reizexpositionsmessungen und den Fragebogenantworten der letzten 10-100 Tage.

5. Verfahren nach einem der vorhergehenden Ansprüche, wobei die Abschätzung des Rückfallrisikos ferner auf mindestens einem der folgenden Faktoren basiert:

   • Teilnahme der betreffenden Person an einem Unterstützungs- oder Behandlungsprogramm im Zusammenhang mit Suchtverhalten;
   • kurz-, mittel- und langfristige Risikofaktoren, darunter mindestens einer der folgenden: Tagesstimmung, Ausübung von Routinen wie Arbeits-, Schul- und Therapieroutinen, Motivation, Essgewohnheiten, Schlafgewohnheiten, Engagement und Selbstwirksamkeit, wie im Fragebogen erfasst; und
   • motorische Informationen darüber, wie der Fragebogen beantwortet wurde.

6. Verfahren nach einem der Ansprüche 1-5, wobei der Reiz Alkohol ist.

7. Verfahren nach einem der Ansprüche 1-5, wobei der Reiz Glücksspiel oder Gaming ist.

8. Verfahren nach einem der Ansprüche 1-5, wobei der Reiz Nahrung ist.

9. Servereinheit (301), konfiguriert zur Abschätzung des Rückfallrisikos einer Person in ihr Suchtverhalten, wobei das Suchtverhalten mit der Exposition gegenüber einem Suchtreiz in Zusammenhang steht, wobei die Servereinheit (301) für Folgendes konfiguriert ist:
Zu mehreren Zeitpunkten wiederholt

   • eine Person aufzufordern, mindestens eines der folgenden Dinge zu tun:

      ◦ eine Messung der Exposition der Person gegenüber dem Reiz durchzuführen;
      ◦ einen Fragebogen zu beantworten, der in einem Messgerät (303) bereitgestellt wird;

   • ein Ergebnis der Messung der Reizexposition (sofern durchgeführt) und der Antworten auf den Fragebogen (sofern erfasst) vom Messgerät (303) zu erhalten;
   • das erhaltene Ergebnis zu speichern;
   • nach Erhalt des Ergebnisses das Rückfallrisiko basierend auf einer Kombination aus dem erhaltenen Ergebnis und historischen Ergebnissen sowie mindestens einem der folgenden Punkte abzuschätzen:

      ◦ Tageszeit der Messung der Reizexposition der betreffenden Person;
      ◦ Wochentag der Messung der Reizexposition der betreffenden Person;
      ◦ Ergebnis der vorherigen Messung der Reizexposition der betreffenden Person;
      ◦ Verstrichene Zeit seit der vorherigen Messung der Reizexposition der betreffenden Person;
      ◦ Anzahl der verpassten historischen Messungen der Reizexposition der betreffenden Person;

   • Bestimmung eines nächsten Zeitpunkts für die Aufforderung an die betreffende Person, basierend auf dem geschätzten Risiko mindestens eine Messung der Reizexposition der betreffenden Person durchzuführen und einen Fragebogen zu beantworten, wobei der nächste Zeitpunkt so bestimmt wird, dass ein Zeitverlauf der Reizexpositionsdaten erfasst wird, der Beginnmuster, Häufigkeit, Dauer und Intensität der Reizexposition aufzeigt; und
   • falls das geschätzte Risiko einen vordefinierten Wert überschreitet, Übermittlung einer Nachricht an einen Dritten (304), wobei dieser Dritte mindestens einer der folgenden sein kann:

- ◦ einen Gesundheitsdienstleister;
- ◦ ein Familienmitglied oder naher Verwandter;
- ◦ ein Arbeitgeber;
- ◦ eine Strafverfolgungsbehörde; und
- ◦ eine staatliche Institution.

10. Servereinheit (301) nach Anspruch 9, wobei die Servereinheit (301) so konfiguriert ist, dass sie das Rückfallrisiko anhand einer Kombination aus Folgendem abschätzt:

- historischen Ergebnissen aus mindestens einer der Reizexpositionsmessungen und den Antworten auf den Fragebogen der letzten 1-30 Tage, und
- historischen Ergebnissen aus mindestens einer der Reizexpositionsmessungen und den Antworten auf den Fragebogen der letzten 10-100 Tage.

11. Servereinheit (301) nach Anspruch 9 oder 10, wobei die Servereinheit (301) ferner so konfiguriert ist, dass sie das Rückfallrisiko anhand mindestens eines der Folgenden abschätzt:

- Teilnahme der Person an einem Unterstützungs- oder Behandlungsprogramm im Zusammenhang mit Suchtverhalten;
- kurz-, mittel- und langfristigen Risikofaktoren, darunter mindestens einer der folgenden Faktoren: Tagesstimmung, Ausübung von Routinen wie Arbeits-, Schul- und Therapieroutinen, Motivation, Essgewohnheiten, Schlafgewohnheiten, Engagement und Selbstwirksamkeit, wie im Fragebogen bewertet; und
- motorische Informationen darüber, wie der Fragebogen beantwortet wurde.

12. System, das so konfiguriert ist, dass es das Risiko eines Rückfalls eines Suchtverhaltens eines Individuums abschätzt, wobei das süchtige Verhalten mit der Exposition gegenüber einem Suchtreiz in Beziehung steht, wobei das System eine Servereinheit (301) nach einem der Ansprüche 9 bis 11 und eine Messvorrichtung (303) umfasst, die für Folgendes konfiguriert ist:
Zu mehreren Zeitpunkten

- mindestens eines der folgenden Verfahren durchzuführen:

  - ◦ Messung der Exposition der Person gegenüber dem Reiz;
  - ◦ Bereitstellung eines Fragebogens für die Person und Erfassung ihrer Antworten;

- Übermittlung des Ergebnisses der Messung der Reizexposition (sofern durchgeführt), und der Fragebogenantworten (sofern erfasst), an eine zentrale Servereinheit (301), um die Abschätzung des Rückfallrisikos zu ermöglichen,

und wobei das Messgerät (303) ferner so konfiguriert ist, dass es die Identifizierung der Person während der Messung ihrer Exposition gegenüber dem Reiz ermöglicht.

13. System nach Anspruch 12, wobei das Messgerät (303) mindestens eines der folgenden umfasst:

- ein Alkoholtestgerät zur Messung des Alkoholgehalts in der Atemluft der betreffenden Person;
- einen Bewegungssensor zur Messung der Bewegung der betreffenden Person;
- einen Pulssensor zur Messung des Pulses der betreffenden Person;
- eine Waage zur Messung des Gewichts der betreffenden Person.
- ein Smartphone zur Messung mindestens eines der folgenden Elemente:

  - ◦ Stellen von Fragen, Sammeln von Antworten und Kommunizieren mit einer zentralen Servereinheit (301);
  - ◦ Überwachen der Online-Glücksspiele oder -Spiele der betreffenden Person.

14. Computerprogrammprodukt, bestehend aus einem computerlesbaren Medium, auf dem ein Computerprogramm mit Anweisungen gespeichert ist, die bei der bei Ausführung durch mindestens einen Prozessor die Ausführung der Schritte des Verfahrens nach einem der Ansprüche 1 bis 8 veranlassen.

**Revendications**

1. Procédé permettant d'estimer le risque de rechute d'un comportement addictif chez un individu, ledit comportement addictif étant lié à l'exposition à un stimulus addictif, ledit procédé comprenant :
à plusieurs moments, de manière répétée

    • la demande (S10), par une unité centrale (301), audit individu d'effectuer au moins l'une des actions suivantes :

        ◦ la réalisation d'une mesure de l'exposition dudit individu audit stimulus ;
        ◦ la réponse à un questionnaire fourni dans un dispositif de mesure (303) ;

    • l'exécution d'au moins l'une des opérations suivantes :

        ◦ la mesure (S20), à l'aide dudit dispositif de mesure (303), de l'exposition dudit individu audit stimulus ;
        ◦ la collecte (S30), à l'aide dudit dispositif de mesure (303), des réponses dudit individu au questionnaire ;

    • la transmission (S40) d'un résultat provenant de ladite mesure d'exposition au stimulus, si elle a été effectuée, et desdites réponses au questionnaire, si elles ont été collectées, depuis ledit dispositif de mesure (303) vers ladite unité centrale de serveur (301) ;
    • le stockage (S50) dudit résultat dans ladite unité centrale de serveur (301) ;
    • lorsque ladite unité centrale de serveur (301) reçoit ledit résultat, ladite unité centrale de serveur (301) estime (S60) un risque de rechute sur la base d'une combinaison du résultat reçu et des résultats historiques, et d'au moins l'un des éléments suivants :

        ◦ l'heure de la journée à laquelle l'exposition de ladite personne audit stimulus a été mesurée ;
        ◦ le jour de la semaine auquel l'exposition de ladite personne audit stimulus a été mesurée ;
        ◦ le résultat de la mesure précédente de l'exposition de ladite personne audit stimulus ;
        ◦ le temps écoulé depuis la mesure précédente de l'exposition de ladite personne audit stimulus ;
        ◦ le nombre de mesures historiques de l'exposition de ladite personne audit stimulus qui ont été manquées ;

    • la détermination (S70), à l'aide de ladite unité serveur centrale (301), d'un moment suivant pour demander à ladite personne d'effectuer au moins l'une des opérations suivantes : la réalisation d'une mesure de l'exposition de ladite personne audit stimulus et la réponse à un questionnaire, sur la base dudit risque estimé, dans lequel le moment suivant est déterminé de manière à collecter une chronologie des données d'exposition aux stimuli qui révèle les schémas d'apparition, la fréquence, la durée et l'intensité de l'exposition aux stimuli ; et
    • si ledit risque estimé est supérieur à un niveau prédéfini, la transmission (S80), depuis ladite unité centrale de serveur (301), d'un message à un tiers (304), dans lequel ledit tiers est au moins l'un des suivants :

        ◦ un prestataire de soins de santé ;
        ◦ un membre de la famille ou un proche parent ;
        ◦ un employeur ;
        ◦ un organisme chargé de l'application de la loi ; et
        ◦ une institution gouvernementale.

2. Procédé selon la revendication 1, comprenant en outre, si ledit risque estimé est supérieur à un niveau prédéfini, la fourniture d'un retour d'information à ladite personne en transmettant (S90) un message à ladite personne.

3. Procédé selon la revendication 1 ou la revendication 2, comprenant en outre l'identification de ladite personne pendant la mesure de l'exposition de ladite personne audit stimulus.

4. Procédé selon l'une quelconque des revendications précédentes, dans lequel ladite estimation d'un risque de rechute est basée sur une combinaison :

    • des résultats historiques d'au moins une desdites mesures d'exposition au stimulus et desdites réponses au questionnaire couvrant les 1 à 30 derniers jours, et
    • des résultats historiques d'au moins une desdites mesures d'exposition au stimulus et desdites réponses au questionnaire couvrant les 10 à 100 derniers jours.

5. Procédé selon l'une quelconque des revendications précédentes, dans lequel ladite estimation d'un risque de rechute est en outre basée sur au moins l'un des éléments suivants :

• la participation de ladite personne à un programme de soutien ou à un programme de traitement lié à un comportement addictif ;
• des facteurs de risque à court, moyen et long terme, comprenant au moins l'un des éléments suivants : l'humeur quotidienne, l'exercice de routines telles que les routines professionnelles, scolaires et thérapeutiques, la motivation, les habitudes alimentaires, les habitudes de sommeil, l'engagement et l'auto-efficacité tels qu'évalués dans ledit questionnaire ; et
• des informations motrices sur la manière dont les réponses au questionnaire ont été fournies.

6. Procédé selon l'une quelconque des revendications 1 à 5, dans lequel ledit stimulus est l'alcool.

7. Procédé selon l'une quelconque des revendications 1 à 5, dans lequel ledit stimulus est les jeux d'argent ou les jeux en ligne.

8. Procédé selon l'une quelconque des revendications 1 à 5, dans lequel ledit stimulus est un aliment.

9. Unité serveur (301) configurée pour estimer un risque de rechute d'un comportement addictif d'un individu, ledit comportement addictif étant lié à l'exposition à un stimulus addictif, dans laquelle l'unité serveur (301) est configurée pour :
à plusieurs moments, de manière répétée

• demander à un individu de faire au moins l'une des choses suivantes :

◦ la réalisation d'une mesure de l'exposition dudit individu audit stimulus ;
◦ la réponse à un questionnaire fourni dans un dispositif de mesure (303) ;

• recevoir un résultat de ladite mesure d'exposition au stimulus, si elle a été effectuée, et desdites réponses au questionnaire, si elles ont été recueillies, à partir dudit dispositif de mesure (303) ;
• stocker ledit résultat reçu ;
• à la réception dudit résultat, estimer un risque de rechute sur la base d'une combinaison du résultat reçu et des résultats historiques, et d'au moins l'un des éléments suivants :

◦ l'heure de la journée à laquelle l'exposition de ladite personne audit stimulus a été mesurée ;
◦ le jour de la semaine auquel l'exposition de ladite personne audit stimulus a été mesurée ;
◦ le résultat de la mesure précédente de l'exposition de ladite personne audit stimulus ;
◦ le temps écoulé depuis la mesure précédente de l'exposition de ladite personne audit stimulus ;
◦ le nombre de mesures historiques de l'exposition de ladite personne audit stimulus qui ont été manquées ;

• déterminer un prochain moment pour demander à ladite personne d'effectuer au moins l'une des opérations consistant à mesurer l'exposition de ladite personne audit stimulus et à répondre à un questionnaire, sur la base dudit risque estimé, dans lequel le prochain moment est déterminé de manière à collecter une chronologie des données d'exposition aux stimuli qui révèle les schémas d'apparition, la fréquence, la durée et l'intensité de l'exposition aux stimuli ; et
• si ledit risque estimé est supérieur à un niveau prédéfini, transmettre un message à un tiers (304), dans lequel ledit tiers est au moins l'un des suivants :

◦ un prestataire de soins de santé ;
◦ un membre de la famille ou un proche parent ;
◦ un employeur ;
◦ un organisme chargé de l'application de la loi ; et
◦ une institution gouvernementale.

10. Unité serveur (301) selon la revendication 9, dans laquelle l'unité serveur (301) est configurée pour estimer un risque de rechute sur la base d'une combinaison :

• de résultats historiques provenant d'au moins une desdites mesures d'exposition à des stimuli et desdites réponses au questionnaire couvrant les 1 à 30 derniers jours, et
• de résultats historiques provenant d'au moins une desdites mesures d'exposition à des stimuli et desdites réponses au questionnaire couvrant les 10 à 100 derniers jours.

11. Unité serveur (301) selon la revendication 9 ou la revendication 10, dans laquelle l'unité serveur (301) est en outre configurée pour estimer un risque de rechute sur la base d'au moins l'un des éléments suivants :

• la participation de ladite personne à un programme de soutien ou à un programme de traitement lié à un comportement addictif ;
• des facteurs de risque à court, moyen et long terme, comprenant au moins l'un des éléments suivants : l'humeur quotidienne, l'exercice de routines telles que les routines professionnelles, scolaires et thérapeutiques, la motivation, les habitudes alimentaires, les habitudes de sommeil, l'engagement et l'auto-efficacité tels qu'évalués dans ledit questionnaire ; et
• des informations motrices sur la manière dont les réponses au questionnaire ont été fournies.

12. Système configuré pour estimer le risque de rechute d'un comportement addictif d'un individu, ledit comportement addictif étant lié à l'exposition à un stimulus addictif, dans lequel le système comprend une unité serveur (301) de l'une quelconque des revendications 9 à 11, et un dispositif de mesure (303) configuré pour :

à plusieurs moments,

• effectuer au moins l'une des opérations suivantes :

◦ la mesure de l'exposition dudit individu audit stimulus ;
◦ la fourniture d'un questionnaire audit individu et la collecte des réponses dudit individu au questionnaire ;

• transmettre un résultat de ladite mesure d'exposition au stimulus, si elle a été effectuée, et desdites réponses au questionnaire, si elles ont été collectées, à une unité serveur centrale (301) afin de permettre l'estimation d'un risque de rechute,

et dans lequel le dispositif de mesure (303) est en outre configuré pour permettre l'identification dudit individu pendant la mesure de l'exposition dudit individu audit stimulus.

13. Système selon la revendication 12, dans lequel le dispositif de mesure (303) comprend au moins l'un des éléments suivants :

• un éthylotest configuré pour mesurer la teneur en alcool dans l'haleine de ladite personne ;
• un capteur de mouvement configuré pour mesurer les mouvements de ladite personne ;
• un capteur de pouls configuré pour mesurer le pouls de ladite personne ;
• une balance configurée pour mesurer le poids de ladite personne.
• un téléphone mobile intelligent configuré pour au moins l'une des fonctions suivantes :

◦ la présentation de questions, la collecte de réponses et la communication avec une unité centrale (301) ;
◦ la surveillance de la participation de ladite personne à des jeux d'argent ou à des jeux en ligne.

14. Produit logiciel comprenant un support lisible par ordinateur sur lequel est stocké un programme informatique comprenant des instructions qui, lorsqu'elles sont exécutées par au moins un processeur, amènent ledit au moins un processeur à exécuter les étapes du procédé selon l'une quelconque des revendications 1 à 8.

Fig. 1

START

REQUEST INDIVIDUAL TO CONDUCT MEASUREMENT AND/OR ANSWER QUESTIONNAIRE — S10

MEASURE INDIVIDUAL'S EXPOSURE TO STIMULUS AND/OR COLLECT QUESTIONNAIRE ANSWERS — S20/S30

TRANSMIT RESULT TO CENTRAL SERVER — S40

STORE RESULT IN CENTRAL SERVER — S50

ESTIMATE RISK OF RELAPSE BASED ON RECEIVED RESULT + HISTORICAL RESULTS — S60

DETERMINE NEXT POINT IN TIME FOR REQUESTING MEASUREMENT AND/OR ANSWERS BASED ON ESTIMATED RISK — S70

TRANSMIT MESSAGE TO THIRD PARTY IF RISK IS GREATER THAN A PREDEFINED LEVEL — S80

END

*Fig. 2a*

S80

TRANSMIT MESSAGE TO INDIVIDUAL IF RISK IS
GREATER THAN A PREDEFINED LEVEL

S90

ACTIVATE ACTUATOR IF RISK IS GREATER THAN A
PREDEFINED LEVEL

S100

END

*Fig. 2b*

*Fig. 3*

*Fig. 4*

*Fig. 5*

*Fig. 6*

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- UA 79063

- WO 2015021042 A1

**Non-patent literature cited in the description**

- **HODGINS, DAVID C.** ; **EL-GUEBALY, NADY**. Retrospective and Prospective Reports of Precipitants to Relapse in Pathological Gambling. *Journal of Consulting and Clinical Psychology*, February 2004, vol. 72 (1), 72-80, http://dx.doi.org/10.1037/0022-006X.72.1.72.
- **CHIH**. Predictive Modeling of Addiction Lapses in a Mobile Health Application. *J Subst Abuse Treat.*, January 2014, vol. 46 (1), 29-35
- **LEUNG**. Effect of self-administered auricular acupressure on smoking cessation--a pilot study. *BMC Complement Altern Med.*, 28 February 2012, vol. 12, 11
- **RH MOOS** ; **BS MOOS**. Rates and predictors of relapse after natural and treated remission from alcohol use disorders. *Addiction.*, 2006, vol. 101 (2), 212-222

- **HODGINS, DAVID C.** ; **GUEBALY, NADY**. Retrospective and Prospective Reports of Precipitants to Relapse in Pathological Gambling. *Journal of Consulting and Clinical Psychology*, February 2004, vol. 72 (1), 72-80, http://dx.doi.org/10.1037/0022-006X.72.1.72
- **HELANDER**. Monitoring of the alcohol biomarkers PEth, CDT and EtG/EtS in an outpatient treatment setting. *Alcohol Alcohol.*, September 2012, vol. 47 (5), 552-7
- **MICHEL** ; **MAKOWSKI**. Comparison of Statistical Models for Analyzing Wheat Yield Time Series. *PLoS ONE*, vol. 8 (10), e78615